# EUROPEAN PATENT APPLICATION

(11) **EP 4 209 192 A1**
(43) Date of publication of application: **12.07.2023**
(21) Application number: 20952586.4
(22) Date of filing: 04.11.2020
(51) Int. Cl.: A61B 18/20, A61N 5/06, A61B 18/00, A61N 5/067

(54) **LIGHT IRRADIATION DEVICE AND IRRADIATION METHOD**

(30) Priority: 02.09.2020 KR 20200111908
(71) Applicant: Wizmedi Co., Ltd., Gyeongsan-si, Gyeongsangbuk-do 38541 (KR)
(72) Inventor: CHOI, Yong Hun, Daegu 42017 (KR); HAN, Yun Soo, Pohang-si, Gyeongsangbuk-do 37655 (KR); HWANG, Je Wan, Seoul 02513 (KR)
(74) Representative: Katérle, Axel
(86) International application number: PCT/KR2020/015339
(87) International publication number: WO 2022/050488

(57) **Abstract**

According to the present disclosure, a radiating apparatus is provided. The radiating apparatus includes a generator for generating perforating light for forming a hole on the skin and a radiating unit for radiating the perforating light to the skin, and the radiating unit is capable of radiating the perforating light multiple times to a target point set on the skin.

## Description

### [Technical Field]

The present disclosure relates to an apparatus and method for radiating light such as a laser to form a hole on the skin.

### [Background Art]

It is necessary to form holes on the skin in order to effectively absorb drugs for treatment of skin diseases or skin care into the skin.

A needle, laser, etc. may be used to form a hole on the skin.

In the case of perforation of the skin using a needle, a high skill level of the operator is required, and there is a problem of causing severe pain.

Compared to general beams, lasers do not diverge and go straight, and have the characteristic of producing strong output in a short time with a single wavelength. Such lasers are non-ionizing beams capable of producing high output, and are highly monochromatic and non-dispersive. Accordingly, when a laser beam is absorbed into skin tissue, etc., exothermic action and photochemical changes occur, resulting in deformation of the skin.

### [Disclosure]

### [Technical Problem]

The present disclosure is to provide an apparatus and method for radiating light that target the upper dermis layer with a depth of approximately 300 *µm* from the surface of the skin.

### [Technical Solution]

The radiating apparatus according to the present disclosure may include a generator for generating perforating light to form a hole on the skin and a radiating unit for radiating the perforating light to the skin, and the radiating unit may radiate the perforating light multiple times to a target point set on the skin.

The radiating method according to the present disclosure may involve an aiming step of aiming radiated light at the skin; a first radiating step of radiating perforating light for forming a hole on the skin toward a first target point among a plurality of target points in a set area formed based on the radiated light; and a second radiating step of radiating the perforating light toward a second target point in the set area after finishing radiating the perforating light to the first target point.

The operation in the second radiating step may be performed after the operation in the first radiating step is fully completed.

In the first radiating step, the perforating light may be intermittently radiated to the first target point for a first set time.

In the radiating method according to the present disclosure, when perforating light is radiated to a plurality of target points in a set area formed based on light radiated to the skin, a first cycle operation in which the perforating light is no longer radiated for a second time after the perforating light is radiated for a first time may be repeated as many times as set for a specific target point on the skin.

In the radiating method according to the present disclosure, after the first cycle operation is repeated the set number of times for the specific target point, another target point in the set area may be automatically selected.

In the radiating method according to the present disclosure, the first cycle operation may be repeated a set number of times for the selected other target point.

In the radiating method according to the present disclosure, the set area may be fixed until the first cycle operation is performed one or more times for each of the plurality of target points in the set area.

The radiating method according to the present disclosure may involve determining the interval and energy of a first laser, seeking the first laser having a shorter interval and stronger energy than a second laser targeting the lower dermal layer and radiating the first laser multiple times to target the upper dermal layer.

### [Advantageous Effects]

By the radiating apparatus and method according to the present disclosure, it may be possible to target the upper dermal layer, which has been difficult to realize with the conventional laser technology.

According to the present disclosure, in particular, it may be possible to induce exothermic action and photochemical changes in the upper dermal layer with a depth of approximately 300 *µm* from the surface of the skin by using a CO2 laser. Through these changes, it may be possible to form a hole extending from the surface of the skin into the upper dermal layer. Through the hole, medical or cosmetic drugs penetrate into the upper dermal layer, and can be directly absorbed into the upper dermal layer or cause various effects.

In addition, by the radiating apparatus and method according to the present disclosure, it may be possible to alleviate various side effects because a laser having energy that could damage the skin is radiated to the skin multiple times in short bursts.

According to the present disclosure, since energy may be intensively delivered only to target tissue while reducing thermal damage to surrounding tissue as much as possible in order to deform the upper dermal layer or form a hole in the upper dermal layer, side effects such as burns and pain are rarely caused. Furthermore, compared to the existing method in which the lower dermal layer is targeted, unnecessary skin irritation is minimized so that the time for skin recovery may be greatly shortened.

### [Description of Drawings]

FIG. 1 is a perspective view showing a radiating apparatus according to the present disclosure.
FIG. 2 is a block diagram showing the radiating apparatus according to the present disclosure.
FIG. 3 is a schematic view showing a cross section of the skin.
FIG. 4 is a schematic view showing a laser according to the comparative embodiment.
FIG. 5 is a schematic view showing perforating light according to the present disclosure.
FIG. 6 is a schematic view showing a first set area.
FIG. 7 is a schematic view showing a second set area.
FIG. 8 is a schematic view showing a third set area.
FIG. 9 is a schematic view showing a plurality of target points formed in a set area.
FIG. 10 is a schematic view showing the operation of a radiating unit.
FIG. 11 is a flowchart illustrating a radiating method according to the present disclosure.
FIG. 12 is a view illustrating a computing device according to an embodiment of the present disclosure.

### [Mode for Disclosure]

Hereinafter, with reference to the accompanying drawings, the embodiments of the present disclosure will be described in detail so that a person having ordinary skill in the technical field to which the present disclosure pertains can easily carry out the embodiments. However, the present disclosure may be embodied in various different forms and is not limited to the embodiments described herein. In addition, in order to clearly describe the present disclosure, parts irrelevant to the description have been omitted from the drawings, and similar reference numerals have been assigned to similar parts throughout the present disclosure.

In the present disclosure, descriptions of the same components will not be repeatedly provided.

In addition, in the present disclosure, when a component is described as being "connected" to another component, it should be understood that the component may be directly connected to the other component or other components may be present therebetween. In contrast, in the present disclosure, when a component is described as being "directly connected" to another component, it should be understood that no other component exists therebetween.

Furthermore, terms used in the present disclosure are only for describing specific embodiments, and are not intended to limit the present disclosure.

In the present disclosure, expressions in the singular form may include the meaning of the plural form unless they clearly mean otherwise in the context.

In the present disclosure, expressions such as "include" or "have" are only for indicating the existence of a feature, number, step, operation, component, part, or combination thereof described in the present disclosure, and are not intended to exclude in advance the existence or the possibility of the addition of one or more other features, numbers, steps, operations, components, parts, or combinations thereof.

In the present disclosure, the expression "and/or" means a combination of a plurality of listed items or any one of the plurality of listed items. In the present disclosure, "A or B" means "A," "B," or "both A and B."

In the present disclosure, detailed descriptions of well-known functions and features that may obscure the gist of the present disclosure will not be provided.

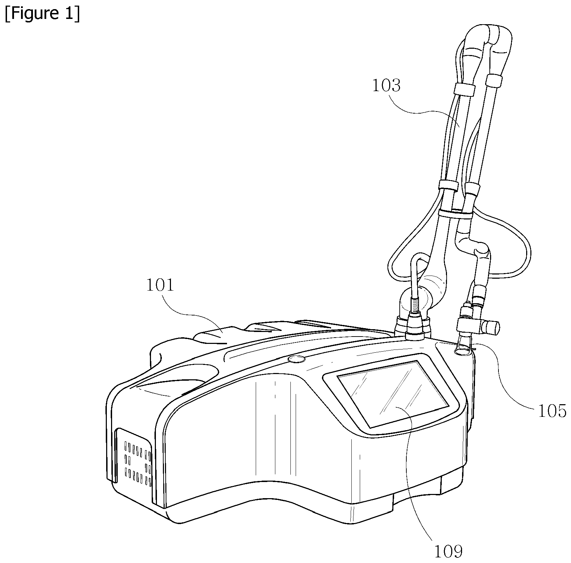

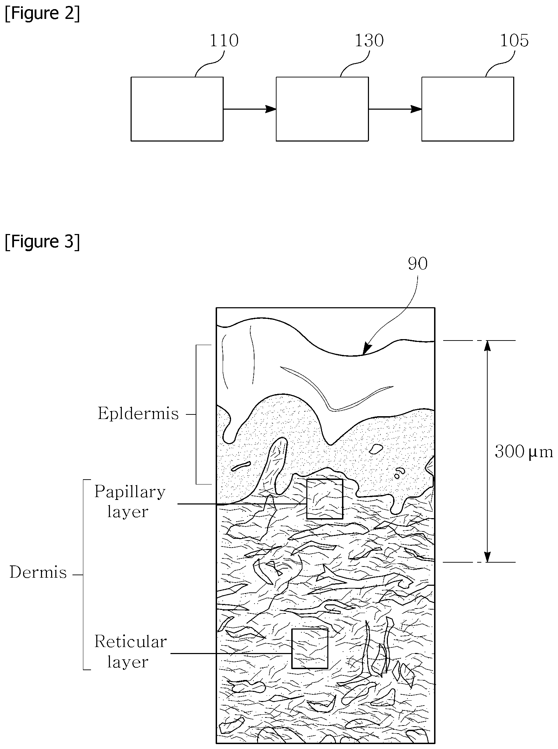

FIG. 1 is a perspective view showing a radiating apparatus according to the present disclosure. FIG.2 is a block diagram showing the radiating apparatus according to the present disclosure.

The radiating apparatus according to the present disclosure may include a tubular body 101, a generator 130 mounted on the body 101, an interface unit 109 and a trigger unit 110 installed on the body 101, an arm 103 rotatably connected to the body 101, and a radiating unit 105 installed at an end of the arm 103.

The body 101 may be formed in various three-dimensional shapes such as pillars and boxes, and an empty space in which the generator 130, etc. can be mounted may be formed inside the case exposed to the outside.

The generator130 may generate perforating light for forming a hole on the skin 90. For example, the perforating light may include a carbon dioxide (CO2) laser, etc.

The CO2 laser is a typical gas laser that oscillates in the infrared area around 9.2 *µ*m and 10.8 *µ*m by obtaining a density inversion between vibrational levels of gaseous carbon dioxide. The CO2 laser was invented in 1964 by Chandra Kumar Patel of Bell Laboratories in the United States, and is so efficient that powers of several kilowatts can be easily obtained. Until now, the CO2 laser is widely used as a high-power laser providing an area of 9.4 *µ*m and 10.6 *µ*m in the industry and the medical field.

The interface unit 109 may be installed on the body 101 and may be exposed to the outside so that a user can watch it or operate it with his/her hands. For example, the interface unit 109 may include a touch screen for displaying various types of information and generating various signals input by the user. In addition, the interface unit 109 may include a display on which various types of information are displayed and means such as buttons for generating various input signals.

The trigger unit 110 may generate a signal for driving the generator 130 or the radiating unit 105 as controlled by a user. The generator 130 or the radiating unit 105 may be driven to radiate perforating light toward the skin by the signal. The trigger unit 110 may be integrally formed with the interface unit 109 or formed separately. For example, the trigger unit 110 may include a pedal that the user controls with his/her feet.

The arm 103 may be formed in a rod shape. When a plurality of arms 103 are provided, the plurality of arms 103 may be connected to move relative to each other by joints.

A waveguide, etc. that guides perforating light generated by the generator 130 to the end of the arm 103 may be formed in the arm 103.

The radiating unit 105 may radiate perforating light to the skin. The way in which the arm 103 is positioned may be adjusted so that the aiming line of the radiating unit 105 is aimed at the target skin.

According to a comparative embodiment that can be assumed, perforating light may be continuously radiated for a long time to deform the skin at a specific target point by exothermic action and photochemical changes, for example, to form a hole on the skin at that point.

The existing skin care or medical skin treatments target the lower dermis layer of the skin. Therefore, means of perforation such as needles, injections, and lasers that pierce the skin to guide various drugs outside the skin to the lower dermis layer have been developed with a focus on forming holes that reach the lower dermis layer.

For example, according to the comparative embodiment mentioned above, the lower dermis layer is targeted in order to stimulate the skin strongly and deeply. However, recent studies and clinical trials have shown that targeting the upper dermis layer is effective for certain skin treatments and skin care.

The dermal layer of the skin consists of two layers: the lower dermis layer (reticular layer) of which fibroblasts produce collagen creating scars to protect the skin and the upper dermis layer (papillary layer) of which fibroblasts repair the skin without creating scars, and targeting the upper dermis layer may be effective for the purpose of skin care.

However, it may be difficult to target the upper dermis layer only by reducing the amount of laser energy continuously radiated as in the comparative embodiment. This is because holes are not formed on the skin at all when the amount of laser energy according to the comparative embodiment is reduced. In contrast, when the amount of laser energy is increased sufficiently to form a hole on the skin, there is a problem in that a hole extending to the lower dermis layer, which was previously targeted, is formed.

Therefore, a new method of forming a hole extending only to the upper dermis layer without penetrating the lower dermis layer is required. For reference, in the present disclosure, the "upper dermis layer" may mean the papillary dermis, and the "lower dermis layer" may mean the reticular dermis.

In order to form a hole extending only to the upper dermis layer without penetrating the lower dermis layer, the radiating unit 105 according to the present disclosure may radiate perforating light to target points set on the skin multiple times.

FIG. 3 is a schematic view showing a cross section of the skin.

There is a dermis layer under the epidermis, and the dermis layer consists of the upper dermis layer (papillary layer) located directly below the epidermis and the lower dermis layer (Reticular layer) located directly below the upper dermis layer.

The radiating apparatus according to the present disclosure may be aimed at creating micropores extending from the outer surface of the epidermis to a depth of less than 300 *µ*m. Compared to the comparative embodiment, the radiating unit 105 according to the present disclosure may increase the peak power and reduce the pulse duration to form micropores having a depth of 300 *µ*m. Through the formation of the micropores, it may be possible to easily send various drugs (effective substances) to the upper dermis layer and minimize thermal damage, side effects, and pain caused in the process of forming the micropores.

The radiating unit 105 may activate fibroblasts by creating micropores using a CO2 laser and mechanically stimulating them so that the production of collagen and elastin may be stimulated and thermal energy may be delivered to the skin, thereby inducing skin regeneration. In addition, the 300 *µ*m-deep holes formed by the radiating unit 105 may be used as a passage through which effective substances are delivered to fibroblasts in the upper dermis layer.

The radiating unit 105 according to the present disclosure may operate in a first mode in which perforating light is intermittently radiated to a specific target point on the skin.

The radiating unit 105 may continue to operate in the first mode without changing a specific target point until the specific target point is hit by perforating light as many times as set in the first mode.

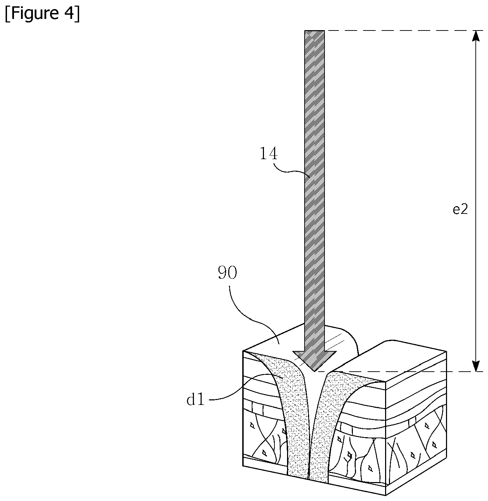

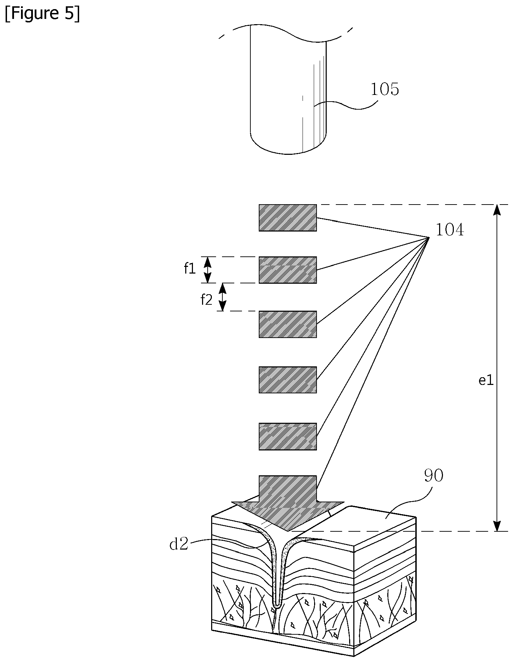

FIG. 4 is a schematic view showing a laser according to the comparative embodiment. FIG. 5 is a schematic view showing perforating light according to the present disclosure.

It may be assumed that a second laser 14 is continuously radiated to a specific target point on the skin with a second amount of energy for a second set time e2 to form a hole extending from the epidermis to the lower dermis layer. Here, the second laser 14 may be a laser according to the comparative embodiment.

The second laser 14 may include a CO2 laser that is continuously radiated without interruption for the second set time e2. By the second laser 14, a hole extending to the lower dermis layer and having a depth of 1 to 2 mm may be formed.

The area d1 of the skin damaged or burned by the second laser 14 may be very large as shown in FIG. 4. Therefore, according to the comparative embodiment, since the area of the damaged skin may be large, pain may be severely induced in the process of forming a hole, and it takes a long time for the skin to recover.

The generator 130 may generate a first laser 104 having a first amount of energy equal to or greater than the second amount, seeking to medically guarantee a patient's safety. Whether a patient's safety is guaranteed may be determined based on an upper limit set to medically prevent burns, etc.

The radiating unit 105 may split a first set time e1 so that a plurality of first periods f1 in which the first laser 104 is radiated and a plurality of second periods f2 in which no laser is radiated are alternately arranged for a specific target point.

The radiating unit 105 may aim at only one specific target point among a plurality of target points for the first set time e1.

The radiating unit 105 may radiate the first laser 104 to a specific target point during each first period f1 of the first set time e1. Here, the time for which the first laser 104 is continuously radiated (duration) may be equal to the duration of the first period f1. The continuous radiating of the first laser 104 may be terminated due to the second period f2 immediately after the first period f1, and the radiating may be resumed in the next first period f1.

The radiating unit 105 may not radiate the first laser 104 to a specific target point in each second period f2 of the first set time e1. According to the present disclosure, a process of radiating and not radiating perforating light to a specific target point may be repeated as many times as set.

The first set time e1 may be 0.5 to 10 times the second set time e2.

The duration of the second period f2 of the first set time e1 may be set to be 5 to 15 times the duration of the first period f1. In this case, the first period f1 may be within a range of 15 *µs* or less. For example, the first period f1 may be within a range of 1 *ns* to 15,000 *ns.*

The first laser 104 radiated by the radiating unit 105 may form a hole not reaching the boundary line between the lower and upper dermis layers.

At least one of the type of perforating light, the first set time e1, how many times the perforating light is radiated to a first target point for the first set time e1, the diameter of the perforating light, the amount of energy of the perforating light, the distance between a plurality of target points in a set area, and the duration of each time in the process of radiating the perforating light to the first target point multiple times for the first set time e1 may be determined seeking to form a hole extending from the epidermis to the upper dermal layer and not reaching the lower dermal layer.

For example, the generator 130 may generate a CO2 laser having a wavelength of 10.6 *µm.*

The radiating unit 105 may radiate a CO2 laser in a first control mode or a second control mode.

Both the first control mode and the second control mode may be operation modes in which a CO2 laser is radiated in the first period f1 and the radiating of the CO2 laser is stopped in the second period f2. However, the first control mode and the second control mode may have a difference in the number of the first period f1 targeting a specific target point.

For example, when a unit period in which one first period f1 and one second period f2 are combined is determined, a first number of times and a second number of times may be set differently.

The first number of times may refer to how many times the unit period is continuously repeated targeting a specific target point in the first control mode. The second number of times may refer to how many times the unit period is continuously repeated targeting a specific target point in the second control mode. When a separate control mode is further required, a third control mode of a third number of times different from the first and second numbers of times, etc. may be added.

The radiating unit 105 may radiate a CO2 laser in any one of the first control mode, the second control mode, and the third control mode. It may be determined by the interface unit 109 which one of the first control mode, the second control mode, and the third control mode is selected.

The position of the end of a hole may be adjusted within the upper dermal layer in the first control mode, the second control mode, or the third control mode. The depth of a hole formed in the first control mode may be the shallowest, and the depth of a hole formed in the third control mode may be the deepest.

According to the present disclosure where the first periods f1 alternate with the second periods f2, perforating light may be briefly radiated to a specific target point a plurality of times as shown in FIG. 5. As a result, as shown in FIG. 5, the area d2 of the skin damaged or burned by the perforating light may be greatly reduced.

Meanwhile, as for the first set time e1 in relation to a specific target point, perforating light may be radiated by the radiating unit 105 during the first period f1, and the perforating light may not be radiated by the radiating unit 105 during the second period f2. In other words, with regard to the specific target point, the second period f2 may correspond to a period of time when the radiating unit 105 remains idle.

In order to speed up skin treatment and the radiating of perforating light, it may be possible to radiate perforating light to another target point using the radiating unit 105 remaining idle during the second period f2 when a specific target point is targeted. According to this embodiment, for another target point, the perforating light may be radiated in each second period f2 of the first set time e1, and the perforating light may not be radiated in each first period f1.

For example, the radiating unit 105 may split the first set time e1 such that the plurality of first periods f1 in which perforating light is radiated and the plurality of second periods in which no perforating light is radiated may be alternated. The radiating unit 105 may radiate perforating light to a first target point on the skin in each first period f1 of the first set time e1. The radiating unit 105 may radiate the perforating light to a second target point on the skin in each second period f2 of the first set time e1 in which no perforating light is radiated to the first target point.

According to this embodiment, the radiating of perforating light to at least two target points may be completed during the first set time e1.

Meanwhile, radiated light may be used to aid a user of a radiating apparatus in aiming the radiating unit 105 at a spot on the skin to be treated.

For example, the radiating unit 105 may radiate light pointing to a target point to the skin. The radiated light may be used for the purpose of visually pointing to the specific point without irritating the skin.

The user may directly see where a spot being targeted is on the skin through the radiated light.

Based on the radiated light, a plurality of target points arranged in a grid may be set within a set area on the skin. The plurality of target points may be made visible on the skin by the radiated light.

The radiating unit 105 may operate in the first mode in which perforating light is intermittently radiated to a specific target point within a set area.

The radiating unit 105 may operate in a second mode where the aiming line of the perforating light is adjusted to aim at another target point within the set area when the operation in the first mode for the specific target point is completed.

When the operation in the second mode is completed, the radiating unit 105 may operate in the first mode for another target point.

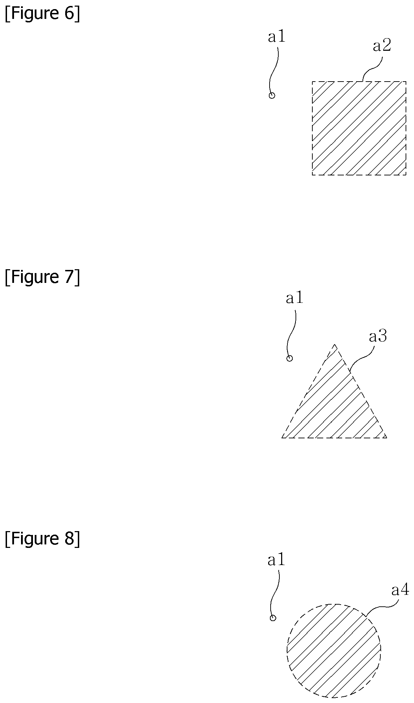

The set area may be formed in various closed curve shapes. FIGS. 6 to 8 show set areas a2, a3, and a4 in different shapes.

FIG. 6 is a schematic view showing a first set area. FIG. 7 is a schematic view showing a second set area. FIG. 8 is a schematic view showing a third set area.

As shown in FIG. 6, the first set area a2 may be formed in a rectangular shape.

As shown in FIG. 7, the second set area a3 may be formed in a triangular shape.

As shown in FIG. 8, the third set area a4 may be formed in a circular shape.

The area of each of the set areas a2, a3, and a4 may be several ten times larger than the diameter of a target point a1. A plurality of target points a1 may be arranged in a set area.

The radiating unit 105 may operate alternately in the first mode and the second mode for a plurality of target points in a set area.

In order to form a hole with a set depth, when the radiating unit 105 completes the operation in the first mode for all target points within a set area, it may operate alternately in the first mode and the second mode for the plurality of target points in the set area over again.

The radiating unit 105 may operate alternately in the first mode and the second mode for a plurality of target points in a set area in order to minimize damage to the skin.

In that case, when the radiating unit 105 completes the operation in the first mode for all the target points within the set area, it may operate alternately in the first mode and the second mode for the plurality of target points in the set area over again.

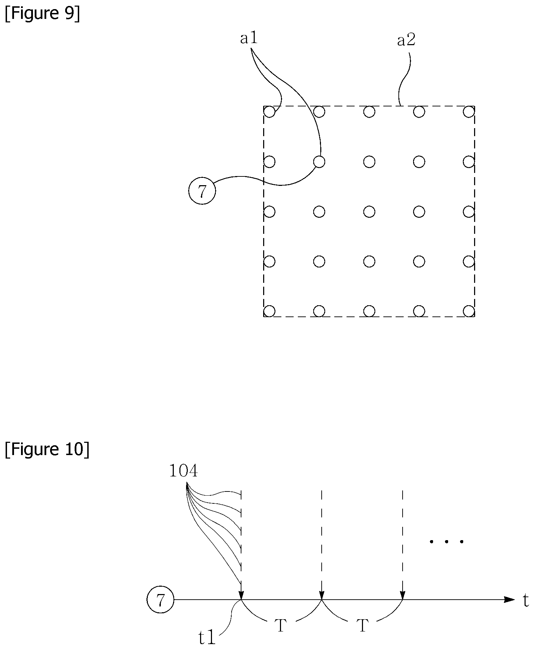

As another example, the radiating unit 105, which may operate in the first mode for a target point other than a specific target point as it does in the second mode, may operate back in the first mode for the specific target point at a set interval T as shown in FIG. 10. According to this embodiment, even before the radiating unit 105 completes the operation in the first mode for all target points in a set area, it may operate back in the first mode for a specific target point. In this case, after the radiating unit 105 operates back in the first mode for the specific target point as many times as set, it may no longer operate back in the first mode for the specific target point.

The radiating unit 105 may radiate perforating light to a plurality of target points formed in a set area on the skin.

The radiating unit 105 may radiate the perforating light to a target point for the first set time e1 and then radiate the perforating light to another target point.

When radiating a first perforating light to each target point, the radiating unit 105 may intermittently radiate the perforating light thereto.

At least one of the amount of energy of the perforating light, the interval at which the perforating light is radiated, how many times the perforating light is radiated, and the first set time e1 may be determined seeking to form a hole extending from the epidermis to the upper dermal layer with a depth of 300 *µm* by intermittently radiating perforating light.

The pattern in which perforating light is radiated in a set area may include a first pattern, a second pattern, a third pattern, and a fourth pattern.

In the first pattern, perforating light may be radiated from the left to the right and from the top to the bottom.

In the second pattern, perforating light may be radiated alternately in a horizontal mode in which it is radiated in one line from the left to the right and in a vertical mode in which it is radiated in one line from the top to the bottom.

In the third pattern, perforating light may be randomly radiated.

The fourth pattern may include a scattering method where target points scattered around a set target point are selected.

Meanwhile, it can be considered relieving pain of a patient using an order in which perforating light is radiated to a plurality of target points formed in a set area.

For example, the radiating unit 105 may set a plurality of target points in a set area on the skin.

The radiating unit 105 may radiate perforating light while selecting a plurality of target points in a first direction. The first direction may refer to an order in which target points are selected in any one of the first pattern, the second pattern, the third pattern, and the fourth pattern.

FIG. 9 is a schematic view showing a plurality of target points formed in a set area.

When a request for a correction to the first direction is input through an input unit, the radiating unit 105 may radiate perforating light while selecting a plurality of target points in a second direction having an opposite tendency to the first direction.

The first direction and the second direction may have opposite tendencies due to at least one of a difference in a distance from a specific target point and a difference in an angle with respect to the center of a set area.

For example, in the case of the first pattern, after the operation in the first mode for a target point at the upper left is completed, the operation in the first mode may be performed for a second target point from the left in the top row.

When a patient complains of pain while performing the operation in the first pattern, it may possible for a user who is treating the patient to select to change the pattern through the interface unit 109. When changing the pattern is selected, the radiating unit 105 may change the order in which target points are selected to operate in the second direction different from the current first direction.

The first direction and the second direction may have opposite tendencies in terms of distance or angle.

For example, when target points are selected one by one from the left to the right in the first direction, a target point farthest from the current target point within a set area may be selected in the second direction. When the second direction according to this embodiment is applied, the operation in the first mode may be performed on a target point at the upper left corner, and then the operation in the first mode may be performed on a target point at the lower right corner.

For example, when target points are selected one by one from the left to the right in the first direction, a target point symmetrical to a target point next to the right may be selected in the second direction. Here, the symmetry may be determined based on the center of a set area. In this case, when the second direction is applied, the operation in the first mode may be performed on a target point at the upper left corner, and then the operation in the first mode may be performed on the second target point from the right in the lower row.

When a request for a re-correction to the first direction is input through an input unit, the radiating unit 105 may radiate perforating light while selecting a plurality of target points in a third direction corresponding to the average of the first direction and the second direction.

For example, when a user's request for a correction to the first direction is received, through the intervention of the second direction, a target point at a position rotated by 180 degrees from a target point to be originally selected based on the center of a set area may be selected as the next target point on which the operation in the first mode will be performed. In this case, when the user's request for a re-correction to the first direction is received, a target point at a position rotated by 90 degrees from a target point to be originally selected may be selected as the next target point on which the operation in the first mode will be performed.

FIG. 10 is a schematic view showing the operation of the radiating unit 105.

There may be provided the trigger unit 110 for generating a driving signal for the generator 130 or the radiating unit 105 as controlled by a user and a locking unit for restricting, upon the operation of the trigger unit 110, the movements of the arm 103 and the radiating unit 105 so that the radiating unit 105 may aim at a fixed position.

When the operation of the trigger unit 110 stops, the locking unit may release the restriction on the movements of the arm 103.

When a specific target point ⑦ is selected as shown in FIG. 10, the radiating apparatus according to the present disclosure may operate in the first mode for the target point ⑦. In this case, in the first mode, perforating light may be intermittently radiated, and the diameter of the perforating light may be approximately 0.2 mm.

In order to ensure that the perforating light that is intermittently radiated multiple times is accurately radiated to the target point ⑦ each time, it may be desirable to fix the radiating unit 105 for the first set time e1 when the operation in the first mode is performed for the target point ⑦.

According to this embodiment, when the trigger unit 110 for controlling the radiating unit 105 to radiate perforating light is operated, the movements of the arm 103 may be restricted by the locking unit. Therefore, according to the present disclosure, since the movements of the radiating unit 105 is restricted by the locking unit while the radiating unit 105 outputs perforating light, during the operation in the first mode, the radiating unit 105 may accurately aim at the target point ⑦ and may radiate the perforating light to the target point ⑦ multiple times.

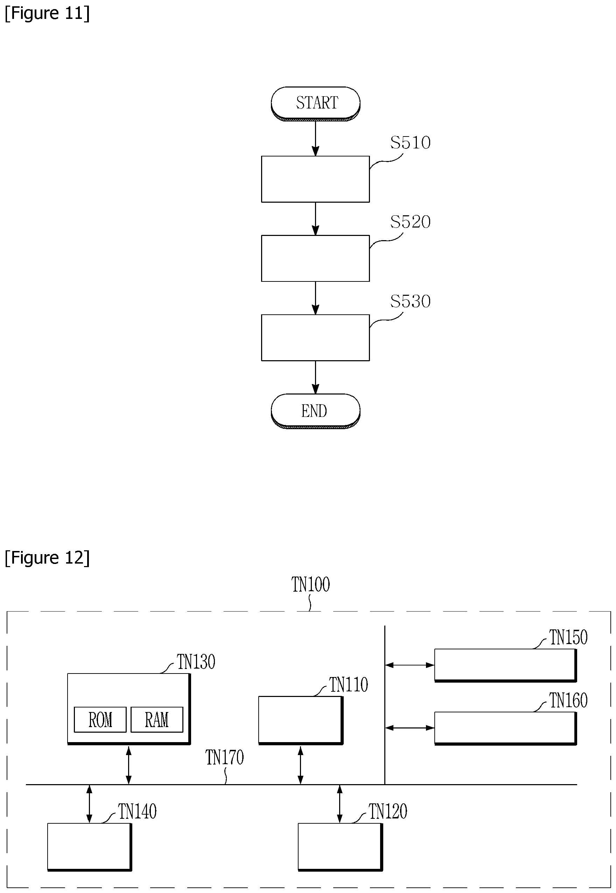

FIG. 11 is a flowchart illustrating a radiating method according to the present disclosure. The radiating method in FIG. 11 may be implemented by the radiating apparatus shown in FIG. 2.

The radiating method shown in FIG. 11 may involve an aiming step (S510), a first radiating step (S520), and a second radiating step (S530).

In the aiming step (S510), radiated light may be aimed at the skin. As an operation performed by the radiating unit 105, a user may specify a position on the skin to which perforating light is to be radiated by using the radiated light.

In the first radiating step (S520), perforating light for piercing the skin may be radiated toward a first target point among a plurality of target points in a set area formed based on the radiated light. This may be an operation performed by the radiating unit 105.

In the second radiating step (S530), when the radiating of the perforating light to the first target point is completed, the perforating light may be radiated toward a second target point in the set area. This may be performed by the radiating unit 105.

The operation in the second radiating step may be performed after the operation in the first radiating step is fully completed.

In the first radiating step, the perforating light may be intermittently radiated to the first target point for the first set time e1.

At least one of the type of perforating light, the first set time e1, how many times the perforating light is radiated to a first target point for the first set time e1, the diameter of the perforating light, the amount of energy of the perforating light, the distance between a plurality of target points in a set area, and the duration of each time in the process of radiating the perforating light to the first target point multiple times for the first set time e1 may be determined seeking to form a hole extending from the epidermis to the upper dermal layer and not reaching the lower dermal layer.

According to the above-described radiating apparatus and radiating method, when perforating light is radiated to a plurality of target points in a set area formed based on light radiated to the skin, the radiating unit 105 may repeatedly perform a first cycle operation for a specific target point on the skin as many times as set.

In that case, the first cycle operation may be initiated at a time point t1 shown in FIG.10. During the first cycle operation, after perforating light is radiated for a first time, no perforating light may be radiated for a second time. From another point of view, the first cycle operation may refer to the operation of the radiating unit 105 during the previously mentioned unit period.

For example, in FIG. 10, the first cycle operation is repeated six times from the time point t1. It may desirable that the first cycle operation is repeated 3 to 12 times.

After the first cycle operation is repeated for a specific target point as many times as set, the radiating unit 105 may automatically select another target point in a set area.

The radiating unit 105 may repeat the first cycle operation for the other selected target point as many times as set.

The locking unit may fix a set area until the first cycle operation is performed one or more times for each of a plurality of target points in the set area.

FIG. 12 is a view illustrating a computing device according to an embodiment of the present disclosure. The computing device TN100 in FIG. 12 may be a device (such as a radiating apparatus) described in the present disclosure.

According to the embodiment in FIG. 12, the computing device TN100 may include at least one processor TN110, a transceiver TN120, and a memory TN130. In addition, the computing device TN100 may further include a storage device TN140, an input interface device TN150, an output interface device TN160, etc. The components included in the computing device TN100 may communicate with each other by being connected by a bus TN170.

The processor TN110 may execute a program command stored in at least one of the memory TN130 and the storage device TN140. The processor TN110 may refer to a central processing unit (CPU), a graphics processing unit (GPU), or a dedicated processor for implementing the methods according to the embodiments of the present disclosure. The processor TN110 may be designed to implement procedures, functions, methods, etc. described in relation to the embodiments of the present disclosure. The processor TN110 may control each component of the computing device TN100.

The memory TN130 and the storage device TN140 may respectively store various information related to the operation of the processor TN110. Each of the memory TN130 and the storage device TN140 may include at least one of a volatile storage medium and a non-volatile storage medium. For example, the memory TN130 may include at least one of a read only memory (ROM) and a random access memory (RAM).

The transceiver TN120 may transmit or receive a wired signal or a wireless signal. The transceiver TN120 may communicate by being connected to a network.

Meanwhile, the embodiments of the present disclosure are not implemented only by the devices and/or methods described above, and may also be implemented through a program for fulfilling functions corresponding to the features of the embodiments of the present disclosure or a recording medium in which the program is recorded. This can be easily done by a person having ordinary skill in the technical field to which the present disclosure belongs by referring to the embodiments described above.

According to the present disclosure, the interval and energy of the first laser may be determined seeking the first laser having a shorter interval and stronger energy than the second laser targeting the lower dermal layer, and the first laser may be radiated multiple times to target the upper dermal layer. According to the present disclosure, it may be possible to minimize damage to the skin in the process of performing procedures for skin treatment and skin care and greatly shorten the time it takes for the skin to recover after the procedure.

After a procedure in which laser was continuously radiated targeting the lower dermal layer (the comparative embodiment) and a procedure in which laser was intermittently radiated targeting the upper dermal layer (the present disclosure) had been respectively tested, the progress of each procedure was monitored.

As a result, it was found that, in the case of the procedure according to the comparative embodiment, the damaged spot on the skin was much larger and the hole on the skin was also much deeper, compared to the procedure according to the present disclosure.

In addition, in the case of the procedure by the radiating apparatus and the radiating method according to the present disclosure, it was observed that scars significantly disappeared one day after the procedure. In contrast, in the case of the procedure according to the comparative embodiment, scars were still clearly visible even seven days after the procedure.

Although the embodiments of the present disclosure have been described in detail above, the scope of the present disclosure is not limited thereto, and various modifications and improvements made by a person having ordinary skill in the art based on the basic concepts of the present disclosure defined in the following claims are also included in the scope of the present disclosure.

## Claims

1. A radiating apparatus comprising:
a generator for generating perforating light to form a hole on the skin; and
a radiating unit for radiating the perforating light to the skin,
wherein the radiating unit radiates the perforating light multiple times to a target point set on the skin.

2. The radiating apparatus of claim 1, wherein the radiating unit operates in a first mode in which the perforating light is intermittently radiated to a specific target point on the skin, and the radiating unit continues to operate in the first mode without changing the specific target point until the specific target point is hit as many times as set by the perforating light in the first mode.

3. The radiating apparatus of claim 1, wherein, assuming a second laser that is continuously radiated to a specific target point on the skin for a second set time with a second amount of energy to form a hole extending from the surface of the skin to the lower dermal layer,
the generator generates a first laser having a first amount of energy equal to or greater than the second amount, seeking to medically guarantee a patient's safety,
the radiating unit splits a first set time so that a plurality of first periods in which the first laser is radiated to the specific target point and a plurality of second periods in which no laser is radiated thereto are alternately arranged,
the radiating unit aims only at the specific target point for the first set time,
the radiating unit radiates the first laser to the specific target point in each first period of the first set time,
the radiating unit does not radiate the first laser to the specific target point in each second period of the first set time,
the first set time is 0.5 to 10 times the second set time,
the duration of the second period is set to be 5 to 15 times the duration of the first period, and
the first laser radiated by the radiating unit forms a hole not reaching the boundary line between the lower dermal layer and the upper dermal layer.

4. The radiating apparatus of claim 1, wherein
the generator generates a CO2 laser,
the radiating unit radiates the CO2 laser in a first control mode or a second control mode,
in both the first control mode and the second control mode, the CO2 laser is radiated in the first period and not radiated in the second period, and
when a unit period is defined as a combination of one first period and one second period, a first number of times, which refers to how many times the unit period is continuously repeated for a specific target point in the first control mode, and a second number of times, which refers to how many times the unit period is continuously repeated for the specific target point in the second control mode, are set to be different from each other.

5. The radiating apparatus of claim 1, wherein the radiating unit splits the first set time so that the plurality of first periods in which the perforating light is radiated and the plurality of second periods in which the perforating light is not radiated are alternately arranged,
the radiating unit radiates the perforating light to a first target point on the skin in each first period of the first set time, and
the radiating unit radiates the perforating light to a second target point on the skin in each second period of the first set time in which no perforating light is radiated to the first target point.

6. The radiating apparatus of claim 1, wherein
the radiating unit radiates light pointing to a target point to the skin,
based on the radiated light, a plurality of target points arranged in a grid in a set area are set on the skin,
the radiating unit operates in the first mode in which the perforating light is intermittently radiated to a specific target point in the set area,
after finishing operating in the first mode for the specific target point, the radiating unit operates in a second mode in which the aiming line of the perforating light is aimed at another target point in the set area, and
after finishing operating in the second mode, the radiating unit operates in the first mode for the other target point.

7. The radiating apparatus of claim 6, wherein
the radiating unit operates alternately in the first mode and the second mode for the plurality of target points in the set area, and
after finishing operating in the first mode for all the target points in the set area, the radiating unit operates alternately in the first mode and the second mode for the plurality of target points in the set area over again.

8. The radiating apparatus of claim 6, wherein the radiating unit, which operates in the first mode for a target point other than the specific target point as it operates in the second mode, operates back in the first mode for the specific target point at a set interval.

9. The radiating apparatus of claim 1, wherein
the radiating unit radiates the perforating light to a plurality of target points formed in a set area on the skin,
the radiating unit radiates the perforating light to each individual target point for the first set time and then radiates the perforating light to another target point,
the radiating unit intermittently radiates the perforating light when radiating a first perforating light to the individual target points, and
at least one of the amount of energy of the perforating light, the interval at which the perforating light is radiated, how many times the perforating light is radiated, and the first set time is determined seeking to form a hole extending from the surface of the skin to the upper dermal layer with a depth of 300 *µm* by intermittently radiating the perforating light.

10. The radiating apparatus of claim 1, wherein
the radiating unit sets a plurality of target points in a set area on the skin,
the radiating unit radiates the perforating light while selecting the plurality of target points in a first direction,
when a request for a correction to the first direction is received through an input unit, the radiating unit radiates the perforating light while selecting the plurality of target points in a second direction having a tendency opposite to the first direction, and
the first direction and the second direction have opposite tendencies due to at least one of a difference in a distance from a specific target point and a difference in an angle with respect to the center of the set area.

11. The radiating apparatus of claim 10, wherein, when a request for a re-correction to the first direction is received through the input unit, the radiating unit radiates the perforating light while selecting a plurality of target points in a third direction corresponding to the average of the first direction and the second direction.

12. The radiating apparatus of claim 1, wherein
a tubular body on which the generator is mounted is provided,
the radiating unit is installed at an end of an arm rotatably connected to the body,
a trigger unit for generating a driving signal for the generator or the radiating unit as controlled by a user and a locking unit for restricting the movements of the arm so that the radiating unit aims at a fixed position upon the operation of the trigger unit are provided, and
the locking unit releases the restriction on the movements of the arm when the operation of the trigger unit stops.

13. A radiating method implemented by a radiating apparatus, comprising:
an aiming step of aiming radiated light at the skin;
a first radiating step of radiating perforating light for forming a hole on the skin toward a first target point among a plurality of target points in a set area formed based on the radiated light; and
a second radiating step of radiating the perforating light toward a second target point in the set area after finishing radiating the perforating light to the first target point,
wherein the operation in the second radiating step is performed after the operation in the first radiating step is fully completed, and, in the first radiating step, the perforating light is intermittently radiated to the first target point for a first set time.

14. The radiating method of claim 13, wherein at least one of the type of the perforating light, the first set time, how many times the perforating light is radiated to the first target point for the first set time, the diameter of the perforating light, the amount of energy of the perforating light, the distance between the plurality of target points in the set area, and the duration of each time in the process of radiating the perforating light to the first target point multiple times for the first set time is determined seeking to form the hole extending from the surface of the skin to the upper dermal layer and not reaching the lower dermal layer.

15. A radiating method implemented by a radiating apparatus, wherein, when perforating light is radiated to a plurality of target points in a set area formed based on light radiated to the skin,
a first cycle operation in which the perforating light is no longer radiated for a second time after the perforating light is radiated for a first time is repeated as many times as set for a specific target point on the skin,
after the first cycle operation is repeated the set number of times for the specific target point, another target point in the set area is automatically selected,
the first cycle operation is repeated a set number of times for the selected other target point, and
the set area is fixed until the first cycle operation is performed one or more times for each of the plurality of target points in the set area.

16. A radiating method implemented by a radiating apparatus, comprising:
determining the interval and energy of a first laser, seeking the first laser having a shorter interval and stronger energy than a second laser targeting the lower dermal layer; and
radiating the first laser multiple times to target the upper dermal layer.
